**Europäisches Patentamt**

**European Patent Office**

**Office européen des brevets**

(11) Numéro de publication: **0 286 524 B1**

## FASCICULE DE BREVET EUROPEEN

(45) Date de publication de fascicule du brevet: **28.10.92**  (51) Int. Cl.⁵: **A61L 2/18**, A61L 2/08

(21) Numéro de dépôt: **88400798.0**

(22) Date de dépôt: **01.04.88**

(54) **Procédé et dispositif de désinfection d'ustensiles.**

(30) Priorité: **07.04.87 FR 8704862**

(43) Date de publication de la demande:
**12.10.88 Bulletin 88/41**

(45) Mention de la délivrance du brevet:
**28.10.92 Bulletin 92/44**

(84) Etats contractants désignés:
**AT BE CH DE ES FR GB GR IT LI LU NL SE**

(56) Documents cités:
**EP-A- 0 196 515**
**WO-A-80/01457**
**FR-A- 2 225 172**
**GB-A- 2 040 150**
**US-A- 4 448 750**

(73) Titulaire: **B.B.C. Société Anonyme**
**47 bis, avenue du Professeur Grasset**
**F-34000 Montpellier(FR)**

(72) Inventeur: **Lerner, Dan**
**Résidence Eden-Parc E Rue du Jeu de Mail**
**des Abbés**
**F-34000 Montpellier(FR)**
Inventeur: **Didier-David, Nadine**
**20, rue des Tamaris**
**F-34970 Lattes(FR)**
Inventeur: **Breux, Jean-Claude**
**20, rue Modigliani**
**F-75015 Paris(FR)**

(74) Mandataire: **Bloch, Gérard et al**
**2, square de l'Avenue du Bois**
**F-75116 Paris(FR)**

## Description

La présente invention a tout d'abord pour objet un procédé de désinfection d'ustensiles dans lequel :
- on dispose lesdits ustensiles dans une cuve contenant un liquide, et
- on soumet ladite cuve à une excitation ultrasonore.

Un tel procédé s'applique au nettoyage des ustensiles médicaux, chirurgicaux, ou encore de laboratoire qui nécessitent une désinfection entre deux utilisations succesives.

On connaît déjà des procédés du type précédent dans lesquels le liquide est de l'eau contenant un solvant par exemple, et les vibrations ultrasonores décollent et dispersent dans le liquide les organismes, comme par exemple les bactéries, qui souillent la surface des ustensiles à nettoyer. Après l'action des ultra-sons, les ustensiles subissent une désinfection de type connu, par exemple par trempage dans un liquide désinfectant, ou par soumission à un rayonnement ultraviolet, ou encore par étuvage. Un tel procédé a pour inconvénients d'une part le fait qu'il est long à mettre en oeuvre, puisqu'il comprend plusieurs étapes successives, et d'autre part le fait que l'efficacité de la désinfection obtenue est parfois insuffisante.

On connaît également un procédé dans lequel le liquide est soumis à un rayonnement ultraviolet en même temps qu'il est soumis à l'excitation ultrasonore. Les bactéries décollées des ustensiles à nettoyer sont détruites lorsqu'elles passent dans une zone soumise au rayonnement ultraviolet, dont les propriétés bactéricides sont bien connues. L'efficacité d'un tel procédé est cependant limitée. En effet, toutes les composantes des rayonnements ultraviolets que l'on sait produire ne sont pas bactéricides. De plus le rayonnement ultraviolet se propageant en ligne droite, il existe, du fait de la présence des ustensiles, des zones d'ombre dans le liquide qui ne sont jamais soumises à l'action du rayonnement. Enfin, le rayonnement ultraviolet est atténué en partie par le liquide. Aussi, afin qu'une bactérie soit détruite à coup sûr, il faut, en pratique, qu'elle soit amenée à passer au voisinage de la surface du liquide. La probabilité d'un tel évènement est donc réduite, et en conséquence l'efficacité de la désinfection.

La présente invention vise à pallier les inconvénients précédents.

A cet effet, elle a pour objet un procédé de désinfection d'ustensiles dans lequel :
- on dispose lesdits ustensiles dans une cuve contenant un liquide, et
- on soumet ladite cuve à une excitation ultrasonore

procédé caractérisé par le fait que :

- ledit liquide est une solution d'au moins un colorant, ayant une longueur d'onde d'absorption comprise entre sensiblement 350 nm et sensiblement 1000 nm,
- au moins pendant ladite excitation ultrasonore, on soumet ladite solution à un éclairement, à au moins ladite longueur d'onde d'absorption, suffisant pour que se produise, au sein de ladite solution, une génération d'oxygène moléculaire activé.

Dans le procédé de l'invention, la destruction des organismes est obtenue, comme on le verra, par l'oxygène moléculaire activé engendré au sein de la solution de colorant, grâce à l'énergie apportée aux molécules de colorant par le rayonnement lumineux auquel est soumise la solution. Ce rayonnement étant dans le spectre visible, il est facile à produire, se propage très bien à l'intérieur du liquide, et engendre de l'oxygène activé même loin de la surface du liquide. L'action des ultrasons favorise le décrochement et la migration des bactéries vers la zone de lumière. De plus, c'est le rayonnement indirect par réflexion simple ou multiple qui atteint les zones qui seraient autrement situées dans l'ombre du rayonnement direct. Dans le cas de bactéries végétatives, on peut ainsi obtenir une désinfection qui est en fait une véritable stérilisation, au sens technique du terme, c'est-à-dire une destruction des bactéries supérieure à 6 logarithmes décimaux. Une destruction à 6 logarithmes décimaux signifie qu'il reste moins d'une bactérie sur $10^6$.

Avantageusement, on chauffe ladite solution à une température comprise entre sensiblement 37°C et sensiblement 40°C.

Selon une caractéristique de l'invention, on ajoute une base à ladite solution pour rendre son pH au moins égal à 9.

On obtient alors une véritable stérilisation, même dans le cas de bactéries sporulées, plus résistantes que les bactéries végétatives.

Selon une autre caractéristique de l'invention, on ajoute à ladite solution au moins un corps choisi parmi les suivants : peroxyde d'hydrogène, un corps susceptible de générer du peroxyde d'hydrogène, et un corps susceptible de générer de l'oxygène actif.

On obtient toujours une véritable stérilisation, même dans le cas de bactéries sporulées, en évitant les inconvénients de l'alcalinisation précédente.

La présente invention a également pour objet un dispositif pour la mise en oeuvre du procédé précédent, caractérisé par le fait qu'il comprend :
- une cuve, prévue pour recevoir des ustensiles à désinfecter et une solution d'au moins un colorant, recouvrant lesdits ustensiles,
- des moyens, réunis à ladite cuve, pour engendrer une vibration ultrasonore et la transmettre à ladite cuve, et

- des moyens pour soumettre, au moins pendant ladite excitation ultrasonore, ladite solution à un éclairement, à au moins la longueur d'onde d'absorption dudit colorant, suffisant pour que se produise, au sein de ladite solution, une génération d'oxygène moléculaire activé.

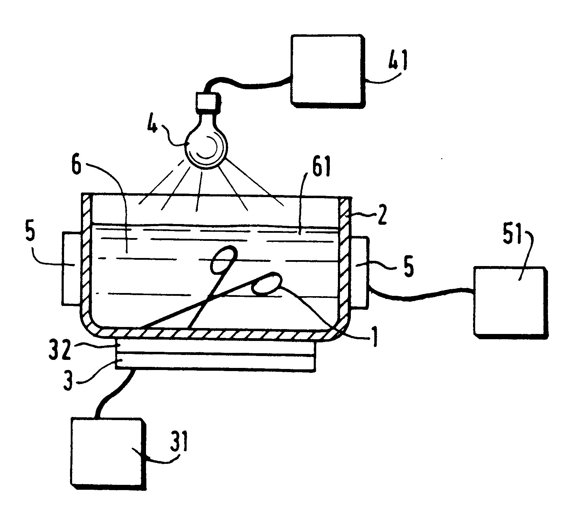

La présente invention sera mieux comprise à la lecture suivante de plusieurs mises en oeuvre du procédé de l'invention, faite en se référant au dessin annexé, sur lequel l'unique figure représente un schéma simplifié, en coupe, du dispositif de l'invention.

Le procédé de désinfection d'ustensiles qui va maintenant être décrit est donc mis en oeuvre à l'aide du dispositif représenté sur la figure.

Ce dispositif comporte une cuve 2, prévue pour recevoir les ustensiles à désinfecter 1, et être remplie par un liquide 6, en quantité suffisante pour recouvrir les ustensiles 1.

La cuve 2 est du type connu à ultrasons, qui comporte par exemple, collé à une rondelle d'adaptation 32 elle-même collée sous le fond de la cuve 2, un transducteur piézo-électrique 3, commandé par un circuit électronique 31. Le transducteur 3 transforme de façon connue le signal de commande du circuit 31 en une vibration ultrasonore transmise à la cuve 2 par la rondelle d'adaptation 32.

Ici, la surface 61 du liquide 6 est éclairée à l'aide d'une source lumineuse, en l'occurrence une lampe 4, reliée à un circuit de commande 41. La lampe 4 est ici une lampe à incandescence de type connu.

Des résistances chauffantes 5, solidaires de la cuve 2, ici par collage sur les parois de la cuve, sont reliées à un circuit électrique de commande 51.

Le liquide 6 est une solution d'au moins un colorant choisi parmi les familles de colorants suivantes : xanthènes (fluorescéine, bleu de méthylène, rhodamine B, proflavine, rose bengale, éosine), porphyrines, phtalocyanines, cyanines, azines et oxazines, quinones, et indigo.

La densité optique de la solution, à la longueur d'onde d'absorption du colorant et pour un trajet de 10 mm, est comprise entre 0,3 et 3. Cette densité optique est mesurée à l'aide d'un appareil de type connu, par exemple un spectrophotomètre à double faisceau comme celui fabriqué par la Société PHILIPS UNICAM.

Lorsque la solution 6 de colorant et les instruments 1 sont disposés dans la cuve 2, on agit sur le circuit 31 pour que la cuve 2 soit soumise à l'excitation ultrasonore du transducteur piézo-électrique 3, et en même temps sur le circuit 41 pour que la surface de la solution 6 soit soumise à l'éclairement dû à la lampe 4.

La durée pendant laquelle la cuve est soumise à l'excitation ultrasonore est comprise entre 10 et 15 minutes, sans que ces valeurs soient critiques.

La durée pendant laquelle la surface de la solution 6 est soumise à l'éclairement de la lampe 4 est au moins égale à celle de l'excitation ultrasonore, mais peut, comme on le verra dans la suite, être plus longue et atteindre sensiblement 40 mn.

Dans ce cas, et comme un homme de métier pourra facilement le comprendre, il peut être avantageux que cette excitation ultrasonore se fasse de façon intermittente, sa durée totale restant du même ordre de grandeur que précédemment.

La lampe 4 est ici une lampe à incandescence de 250 W, disposée à 15 cm de la surface de la solution 6, ce qui correspond sensiblement à un éclairement moyen, sur le spectre visible de 0,5 W/cm$^2$. Il est également possible d'utiliser une lampe pulsée, délivrant de l'énergie lumineuse sous forme d'éclairs. Dans ce cas, cette lampe est réglée pour que l'éclairement soit, en moyenne de 0,5 W/cm$^2$. Naturellement, et comme on peut facilement le comprendre, cette valeur de l'éclairement n'est pas critique, et il est possible de choisir un éclairement de valeur différente, à condition toutefois que celui-ci soit suffisant pour que se produise, au sein de la solution, une génération d'oxygène moléculaire activé.

Cette condition étant remplie, la durée minimale d'éclairement sera d'autant plus faible que l'éclairement sera élevé. Il n'est pas obligatoire que la solution 6 ne soit soumise au rayonnement de la lampe que par sa surface 61, et, dans le cas où la cuve 2 est transparente, on peut prévoir plusieurs sources lumineuses pour éclairer la solution 6 de toutes parts.

La génération d'oxygène moléculaire activé peut être mise en évidence en faisant un des tests de photooxydation connus. On peut citer, par exemple, parmi les tests chimiques, le test à l'α-terpinène qui, en présence d'oxygène activé, donne de l'ascaridole et, parmi les tests physiques, la détection directe spectroscopique de la fluorescence à 1,27 μm.

Par l'expression "oxygène moléculaire activé", on entend de l'oxygène moléculaire dans lequel la répartition des électrons dans la molécule et/ou le nombre des électrons de la molécule sont modifiés. Or, on sait que toutes les propriétés chimiques d'une espèce sont liées à la répartition et/ou au nombre d'électrons de cette espèce.

L'oxygène moléculaire activé qui prend naissance au sein de la solution est obtenu à partir de l'oxygène moléculaire de l'air dissous dans la solution.

Cette activation ne se fait pas par voie directe, mais est réalisée à l'aide d'un médiateur, dit sensibilisateur, en l'occurrence le colorant. En effet, les molécules, ou le mélange de molécules, d'un colorant absorbent la lumière de longueur d'onde égale ou voisine d'une longueur d'onde caractéristique du colorant, dite longueur d'onde du maximum d'absorption. Ces longueurs d'onde d'absorption, comprises ici sensiblement entre 350 nm et 1000 nm, correspondent évidemment à la couleur complémentaire de la couleur sous laquelle apparaît le colorant.

L'énergie lumineuse absorbée par le colorant est convertie en énergie électronique et le sensibilisateur se trouve porté à un état dit électroniquement excité. Cet état est instable et le sensibilisateur cherche à revenir à l'état normal, dit état fondamental, en se débarrassant de son excédent d'énergie.

Cette opération peut prendre deux formes efficaces différentes lorsque l'oxygène moléculaire est considéré :

1) La première forme consiste en un transfert d'énergie électronique vers l'oxygène. Ce dernier se trouve à son tour à un état électronique excité, dit singulet (Cette terminologie vient de la prise en compte par la théorie quantique des propriétés magnétiques des électrons).

Dans cet état singulet, qui correspond à une répartition différente des électrons, l'oxygène possède des propriétés chimiques tout à fait remarquables. C'est un oxydant bien plus puissant qu'à l'état fondamental. Il a de plus une durée de vie exceptionnelle pour une espèce activée : 45 mn en phase gaz diluée et 4 $\mu$sec en solution dans l'eau. Ceci accroît sa probabilité de rencontrer une molécule cible afin de l'oxyder.

2) La deuxième forme consiste en un transfert d'un électron du colorant sur l'oxygène moléculaire. Ce dernier voit le nombre de ses électrons augmenter et il a donc ses propriétés chimiques profondément modifiées. L'espèce obtenue, notée $O_2^{-}$ est connue sous le nom d'oxygène superoxyde. Elle a aussi une réactivité exceptionnelle, mais différente de celle de l'oxygène singulet.

Ces deux phénomènes peuvent être observés simultanément chez un sensibilisateur donné avec des pourcentages qui vont dépendre de l'environnement. Ils peuvent être obtenus avec un mélange de sensibilisateurs, dont certains conduisent à l'oxygène singulet et dont les autres agissent par transfert d'électron (c'est-à-dire à l'oxygène superoxyde).

Les deux espèces d'oxygène activées vont attaquer en particulier toutes les fonctions insaturées des molécules organiques, y compris celles d'origine biologique.

Vis-à-vis des organismes comme les bactéries, levures, spores ou virus, par exemple, qui souillent les instruments 1, cette action se traduit par une atteinte à l'intégrité des membranes protectrices, entraînant la destruction de ces organismes.

Lorsque les ustensiles à désinfecter sont souillés par des bactéries sporulées, on améliore notablement l'efficacité de la désinfection en alcalinisant la solution à l'aide d'une base forte, jusqu'à ce que son pH atteigne une valeur égale ou supérieure à 9 . On peut simultanément chauffer la solution, en commandant les résistances 5, de façon à ce que la température de la solution soit de l'ordre de 37 à 40° C.

Cette opération nécessite cependant un rinçage du matériel, et n'est pas possible si la cuve ou les instruments comprennent des parties en aluminium, en acier non traité, en cuivre, en chrome usagé, ou en laiton. On se trouve en pratique limité aux matériaux inoxydables ou chromés, aux matières plastiques ou au téflon.

Pour éviter les inconvénients précédents et utiliser ainsi tous les matériaux, on peut, au lieu d'alcaliniser la solution 6, y ajouter du peroxyde d'hydrogène, ou un corps susceptible de générer du peroxyde d'hydrogène ou de l'oxygène actif. Parmi les corps susceptibles de générer du peroxyde d'hydrogène ou de l'oxygène actif, il est intéressant d'utiliser des corps sous forme pulvérulente susceptible de générer du peroxyde d'hydrogène dès leur mise en solution. Parmi ces corps, on peut citer les persulfates, l'urée peroxyhydratée, le peroxycarbonate de sodium, et les peroxomonosulfates, par exemple.

Le peroxyde d'hydrogène $H_2O_2$, en se décomposant, libère de grandes quantités d'espèces actives qui ajoutées à l'oxygène moléculaire activé libéré sous l'action de l'éclairement, augmentent l'efficacité de la désinfection, en particulier vis-à-vis des bactéries sporulées.

Des exemples de mise en oeuvre du procédé et les résultats obtenus sont maintenant donnés. Les exemples 3, et 5 à 10, mettent en évidence la véritable stérilisation obtenue dans le cas des bactéries sporulées particulièrement difficiles à détruire avec les procédés de l'art antérieur. Les exemples 8 à 10 montrent la réduction de la durée d'éclairement qui résulte de l'utilisation d'une solution de plusieurs colorants au lieu d'une solution d'un unique colorant.

Exemple 1

La solution de colorant utilisée est une solution de bleu de méthylène, dont la densité optique, mesurée à la longueur d'onde d'absorption, en l'occurrence à 655 nm, est de l'ordre de 2,5.

Cette solution est contaminée par une bactérie végétative Sarcinea Lutea en suspension dans l'eau, à raison de $3,8.10^7$ bactéries par ml.

Cette solution est disposée dans une cuve à ultra-sons du type décrit et soumise à l'éclairement d'une lampe à incandescence de 250 W située à 15 cm de la surface du liquide pendant 15 mn, et à l'action conjuguée des ultra-sons pendant la même durée.

Après ce traitement, plusieurs échantillons de la solution sont ensemencés en milieu nutritif et placés dans un incubateur à 37° pour une durée de 48 heures. Aucune flore microbiologique détectable n'apparaît, ce qui indique une destruction de bactéries supérieure à 6 logarithmes décimaux.

Exemple 2

On utilise la même solution de colorant que dans l'exemple 1.

Au lieu de contaminer cette solution par une bactérie végétative en suspension, comme dans l'exemple 1, on dispose la solution dans la cuve à ultra-sons où on a déposé des tubes de verre ouverts aux deux extrémités. Sur la paroi interne de chaque tube ont été séchés $6,5.10^6$ spores de la bactérie Bacillus Stéarothermophilus ATCC 7593, de type aérobie. Ces tubes, de diamètre 2,5 mm et de longueur 45 mm, simulent bien, du fait de leur forme et de la souillure de leurs parois internes, les ustensiles médicaux et chirurgicaux que le procédé de l'invention permet de nettoyer.

La solution de colorant dans laquelle trempent les tubes est soumise au même éclairement que dans l'exemple 1 pendant 40 mn et à l'action conjuguée des ultra-sons pendant 15 mn.

Les résultats du traitement, obtenus par incubation en milieu nutritif à 37°C pendant 48 heures, indiquent une destruction des spores voisine de 3 logarithmes décimaux.

Exemple 3

On utilise la même solution de colorant que dans l'exemple 1.

Cette solution est contaminée par une bactérie sporulée Bacillus Subtilis ATCC 6633, de type aérobie, en suspension dans l'eau, à raison de $1,9.10^6$ spores par ml.

On ajoute une base forte à cette solution, pour obtenir un pH de l'ordre de 12,7, on la dispose dans la cuve à ultra-sons, on la chauffe à une température de 40°C, et on la soumet au même éclairement que dans l'exemple 1 pendant 35 mn, et à l'action conjuguée des ultra-sons pendant 15 mn.

Les résultats du traitment indiquent une destruction des spores supérieure à 6 logarithmes décimaux.

Exemple 4

On utilise la même solution de colorant que dans l'exemple 1.

Cette solution est contaminée par la bactérie végétative Sarcinea Lutea, comme dans l'exemple 1.

On ajoute une base forte à cette solution pour obtenir un pH de l'ordre de 12,7, on la dispose dans la cuve à ultra-sons et on la soumet au même éclairement que dans l'exemple 1 pendant 10 mn, et à l'action conjuguée des ultra-sons pendant la même durée.

Les résultats du traitement indiquent une destruction des bactéries supérieure à 7 logarithmes décimaux.

Exemple 5

On utilise la même solution de colorant que dans l'exemple 1.

Cette solution est contaminée par la bactérie sporulée Bacillus Subtilis comme dans l'exemple 3.

On ajoute du peroxyde d'hydrogène à 10 volumes à cette solution, on la chauffe à une température de 40°C, et on la soumet au même éclairement que dans l'exemple 1 pendant 40 minutes, et à l'action conjuguée des ultra-sons pendant 15 mn.

Les résultats du traitement indiquent une destruction des spores supérieure à 6 logarithmes décimaux.

Exemple 6

On utilise la même solution de colorant que dans l'exemple 1.

On dispose la solution dans la cuve à ultra-sons où on a déposé des tubes de verre comme dans l'exemple 2. Sur la paroi interne de chaque tube ont été séchés $2,7.10^6$ spores de la bactérie Clostridium Sporogenes C.I.P.7803, de type anaérobie.

On ajoute du peroxyde d'hydrogène à 10 volumes à cette solution, on la chauffe à une température de 40°C, et on la soumet au même éclairement que dans l'exemple 1 pendant 40 minutes, et à l'action conjuguée des ultra-sons pendant 15 mn.

Les résultats du traitement indiquent une destruction des spores supérieure à 6 logarithmes décimaux.

Exemple 7

On utilise la même solution de colorant que dans l'exemple 1.

Cette solution est contaminée par la bactérie sporulée Bacillus Subtilis comme dans l'exemple 3.

On ajoute du persulfate d'ammonium à la solution, et on la soumet au même éclairement que dans l'exemple 1 pendant 40 mn, et à l'action conjuguée des ultra-sons pendant 15 mn.

Les résultats du traitement indiquent une destruction des spores supérieure à 6 logarithmes décimaux.

### Exemple 8

On utilise une solution de deux colorants, en l'occurrence le bleu de méthylène et la proflavine, solution dont la densité optique, mesurée à chacune des longueurs d'onde d'absorption, à savoir 655 nm et 450 nm, respectivement, est de l'ordre de 2,5 et de 1, respectivement.

Cette solution est contaminée par la bactérie sporulée Bacillus Subtilis comme dans l'exemple 3.

On ajoute du peroxyde d'hydrogène à 10 volumes à cette solution, on la chauffe à une température de 40°C, et on la soumet au même éclairement que dans l'exemple 1 pendant 30 mn, et à l'action conjuguée des ultra-sons pendant 15 mn.

Les résultats du traitement indiquent une destruction des spores voisine de 7 logarithmes décimaux.

### Exemple 9

On utilise une solution de trois colorants, en l'occurrence le rose bengale, l'éosine et la rhodamine B, solution dont la densité optique, mesurée à chacune des longueurs d'onde d'absorption, à savoir 548 nm, 520 nm, et 540 nm, respectivement, est de l'ordre de 1,5 , de 1,5 , et de 2, respectivement.

La contamination, le traitement et les résultats sont identiques à ceux de l'exemple 8.

### Exemple 10

On utilise une solution de cinq colorants, en l'occurrence le bleu de méthylène, le rose bengale, l'éosine, la rhodamine B, la proflavine, solution dont la densité optique, mesurée à chacune des longueurs d'onde d'absorption, à savoir 655 nm, 548 nm, 520 nm, 540 nm et 450 nm, respectivement, est de l'ordre de 2,5, de 1,5, de 1,5, de 2, et de 1, respectivement.

La contamination, le traitement et les résultats sont identiques à ceux des examples 8 et 9.

### Revendications

1. Procédé de désinfection d'ustensiles dans lequel :
   - on dispose lesdits ustensiles dans une cuve contenant un liquide, et
   - on soumet ladite cuve à une excitation ultrasonore,

   procédé caractérisé par le fait que :
   - ledit liquide est une solution d'au moins un colorant, ayant une longueur d'onde d'absorption comprise entre sensiblement 350 nm et sensiblement 1000 nm,
   - au moins pendant ladite excitation ultrasonore, on soumet ladite solution à un éclairement à au moins ladite longueur d'onde d'absorption, suffisant pour que se produise, au sein de ladite solution, une génération d'oxygène moléculaire activé.

2. Procédé selon la revendication 1, caractérisé par le fait que l'on chauffe ladite solution à une température comprise entre sensiblement 37°C et sensiblement 40°C.

3. Procédé selon l'une des revendications 1 ou 2, caractérisé par le fait que l'on ajoute une base à ladite solution pour rendre son pH au moins égal à 9.

4. Procédé selon l'une des revendications 1 ou 2, caractérisé par le fait que l'on ajoute à ladite solution au moins un corps choisi parmi les suivants : peroxyde d'hydrogène, un corps susceptible de générer du peroxyde d'hydrogène, et un corps susceptible de générer de l'oxygène actif.

5. Procédé selon l'une des revendications 1 à 4, dans lequel ledit colorant est choisi parmi les familles de colorants suivantes : xanthènes (fluorescéine, bleu de méthylène, rhodamine B, proflavine, rose bengale, éosine), porphyrines, phtalocyanines, cyanines, azines et oxazines, quinones, et indigo.

6. Procédé selon l'une des revendications 1 à 5, dans lequel la valeur de la densité optique de la solution de colorant, mesurée à la longueur d'onde d'absorption est comprise entre sensiblement 0,3 et sensiblement 3.

7. Procédé selon l'une des revendications 1 à 6, dans lequel ledit éclairement étant de 0,5 W/cm$^2$ en moyenne, sa durée est comprise entre sensiblement 10 mn et sensiblement 40 mn.

8. Procédé selon l'une des revendications 1 à 7, dans lequel ladite excitation ultrasonore est de durée totale comprise entre sensiblement 10 mn et sensiblement 15 mn.

9. Dispositif pour la mise en oeuvre du procédé selon l'une des revendications 1 à 8, caractérisé par le fait qu'il comprend :
- une cuve (2), prévue pour recevoir des ustensiles (1) à désinfecter et une solution (6) d'au moins un colorant, recouvrant lesdits ustensiles (1),
- des moyens (3, 31, 32), réunis à ladite cuve (2), pour engendrer une excitation ultrasonore et la transmettre à ladite cuve (2), et
- des moyens (4, 41) pour soumettre, au moins pendant ladite excitation ultrasonore, ladite solution (6) à un éclairement, à au moins la longueur d'onde d'absorption dudit colorant, suffisant pour que se produise, au sein de ladite solution, une génération d'oxygène moléculaire activé.

10. Dispositif selon la revendication 9, comprenant en outre des moyens (5, 51), solidaires de ladite cuve (2), pour chauffer ladite solution (6).

## Claims

1. Method for disinfecting utensils in which :
   - said utensils are disposed in a tank containing a liquid, and
   - said tank is subjected to ultrasonic excitation, characterized in that said liquid is a solution of at least one dye having an absorption wave-length between substantially 350 nm and substantially a 1000 nm,
   - at least during said ultrasonic excitation, said solution is subjected to illumination at said absorption wave-length at least, sufficient for activated molecular oxygen to be generated within said solution.

2. Method as claimed in claim 1, characterized in that said solution is heated to a temperature between substantially 37°C and substantially 40°C.

3. Method as claimed in one of claims 1 or 2, characterized in that a base is added to said solution so as to make its pH at least equal to 9.

4. Method as claimed in one of claims 1 or 2, characterized in that at least one substance is added to said solution chosen from the following : hydrogen peroxide, a substance capable of generating hydrogen peroxide, and a substance capable of generating active oxygen.

5. Method as claimed in one of claims 1 to 4, characterized in that said dye is chosen from the following dye families : xanthenes (fluoresceine, methylene blue, rhodamine B, proflavine, bengal pink, eosine), porphyrines, phtalocyanines, cyanines, azines and oxazines, quinones and indigo.

6. Method as claimed in one of claims 1 to 5, characterized in that the value of the optical density of the dye solution, measured at the absorption wave-length, is between substantially 0.3 ans substantially 3.

7. Method as claimed in one of claims 1 to 6, characterized in that said illumination being $0.5W/cm^2$ on average, its duration is between substantially 10 mn and substantially 40 mn.

8. Method as claimed in one of claims 1 to 7, characterized in that the total time for said ultrasonic excitation is between substantially 10 mn and substantially 15 mn.

9. Device for implementing the method, as claimed in one of claims 1 to 8, characterized in that it comprises :
   - a tank (2), for receiving utensils (1) to be disinfected and a solution (6) of at least one dye, covering said utensils (1),
   - means (3, 31, 32), connected to said tank (2), for generating an ultrasonic excitation and transmitting it to said tank (2), and
   - means (4,41) for subjecting said solution (6), at least during said ultrasonic excitation, to illumination, at least at the absorption wave-lenght of said dye, sufficient for activated molecular oxygen to be generated within said solution.

10. Device as claimed in claim 9, further including means (5,51), fast with said tank (2), for heating said solution (6).

## Patentansprüche

1. Verfahren zur Desinfektion von Instrumenten, bei dem:
   - man die Instrumente in eine Wanne legt, die eine Flüssigkeit enthält, und
   - man diese Wanne einer Ultraschallerregung aussetzt, ein Verfahren, dadurch gekennzeichnet, daß:
   - die Flüssigkeit eine Lösung mindestens eines Farbstoffs ist, der eine Absorptionswellenlänge zwischen im wesentlichen 350 nm und im wesentlichen 1000 nm besitzt,
   - man die Lösung mindestens während der Ultraschallerregung einer Beleuchtung bei zumindest dieser Absorptionswellenlänge aussetzt, deren Stärke ausreicht, um im Innern der Lösung die Entstehung aktivierten molekularen Sauerstoffs zu bewirken.

2. Verfahren nach Anspruch 1, dadurch gekennzeichnet, daß man die Lösung auf eine Temperatur zwischen im wesentlichen 37°C und im wesentlichen 40°C erwärmt.

3. Verfahren nach einem der Ansprüche 1 oder 2, dadurch gekennzeichnet, daß man der Lösung eine Base zusetzt, um ihren pH-Wert auf mindestens 9 zu bringen.

4. Verfahren nach einem der Ansprüche 1 oder 2, dadurch gekennzeichnet, daß man der Lösung mindestens eine Substanz zusetzt, die unter den folgenden gewählt wird: Wasserstoffperoxid, einer Substanz, welche zur Bildung von Wasserstoffperoxid fähig ist, und einer Substanz, welche zur Bildung von aktivem Sauerstoff fähig ist.

5. Verfahren nach einem der Ansprüche 1 bis 4, bei dem der Farbstoff unter den folgenden Farbstoff-Familien gewählt wird: den Xanthenen (Fluorescein, Methylenblau, Rhodamin B, Proflavin, Bengalrosa, Eosin), den Porphyrinen, den Phthalocyaninen, den Cyaninen, den Azinen und Oxazinen, den Chinonen und Indigo.

6. Verfahren nach einem der Ansprüche 1 bis 5, bei dem der Wert der optischen Dichte der Farbstofflösung, gemessen bei der Absorptionswellenlänge, zwischen im wesentlichen 0,3 und im wesentlichen 3 liegt.

7. Verfahren nach einem der Ansprüche 1 bis 6, bei dem die Beleuchtungsstärke im Mittel 0,5 W/cm$^2$ und die Dauer der Beleuchtung zwischen im wesentlichen 10 mn und im wesentlichen 40 mn beträgt.

8. Verfahren nach einem der Ansprüche 1 bis 7, bei dem die Ultraschallerregung eine Gesamtdauer zwischen im wesentlichen 10 mn und im wesentlichen 15 mn besitzt.

9. Vorrichtung zur Durchführung des Verfahrens nach einem der Ansprüche 1 bis 8, dadurch gekennzeichnet, daß sie umfaßt:
   - eine Wanne (2), dazu vorgesehen, die zu desinfizierenden Instrumente (1) und eine Lösung (6) mindestens eines Farbstoffs, welche diese Instrumente (1) bedeckt, aufzunehmen,
   - Mittel (3, 31, 32), die mit der Wanne (2) verbunden sind und die dazu dienen, eine Ultraschallerregung zu erzeugen und sie auf die Wanne (2) zu übertragen, und
   - Mittel (4, 41), um die Lösung (6) mindestens während dieser Ultraschallerregung einer Beleuchtung bei zumindest der Absorptionswellenlänge des Farbstoffs auszusetzen, deren Stärke ausreicht, um im Innern der Lösung die Entstehung aktivierten molekularen Sauerstoffs zu bewirken.

10. Vorrichtung nach Anspruch 9, die außerdem mit der Wanne fest verbundene Mittel (5, 51) zum Erwärmen der Lösung (6) umfaßt.